**Europäisches Patentamt**

**European Patent Office**

**Office européen des brevets**

(19)

(11) Publication number : **0 468 764 A1**

# (12) EUROPEAN PATENT APPLICATION

(21) Application number : **91306739.3**

(22) Date of filing : **24.07.91**

(51) Int. Cl.⁵ : **A61K 37/64,** C07K 15/00,
A61K 35/62

(30) Priority : **24.07.90 US 557278**

(43) Date of publication of application :
**29.01.92 Bulletin 92/05**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Dunwiddie, Christopher**
**510 Remington Court**
**Chalfont, PA 18914 (US)**
Inventor : **Vlasuk, George P.**
**786 Hartley Drive**
**Lansdale, PA 19446 (US)**

(74) Representative : **Barrett-Major, Julie Diane et al**
**Merck & Co., Inc. European Patent Department Terlings Park Eastwick Road Harlow Essex CM20 2QR (GB)**

(54) **Method for administering a therapeutic anticoagulant.**

(57)    A method for inhibiting blood coagulation Factor Xa. The method involves subcutaneous bolus injection of a protein-containing pharmaceutical composition at low dosage to provide long-term effective means for preventing blood coagulation.

EP 0 468 764 A1

## BACKGROUND OF THE INVENTION

Thrombosis is a major cause of mortality in developed countries. Current therapy depends on the use of anticoagulant and thrombolytic agents to prevent and manage non-fatal thrombotic conditions. Heparin, the most widely used anticoagulant, enhances the effect of antithrombin III, thereby blocking thrombin activity (Hemker, International Society on Thrombosis and Haemostasis, Leuven University Press, Leuven (1987)). Its use is often accompanied by complications due to its broad binding specificities (Huisse et al., Thromb. Res. 27 (1982) 485-490).

The role of blood coagulation is to provide an insoluble fibrin matrix for consolidation and stabilization of a hemostatic plug. Formation of a cross-linked fibrin clot results from a series of biochemical interactions involving a range of well-characterized plasma proteins.

The interactions are divided into what are termed the "intrinsic pathway", in which all the substances necessary for fibrin formation are present in precursor form in circulating plasma, and the extrinsic pathway in which thromboplastin, derived from tissues, bypasses several steps in the process and accelerates clot formation. The two pathways are highly interdependent and Factor VII, Factor IX and Factor X are mutually activated (J.C. Giddings "Molecular Genetics and Immuoanalsis in Blood Coagulation" Ellis Horwood Ltd., Chichester, England 1988, p. 17).

The role of Factor X in the coagulation cascade has been reviewed by Zur et al. "Tissue factor pathways of blood coagulation", Haemostasis and Thrombosis, 1st Edition (Bloom et al., Eds) Churchill Livingstone, Edinburgh, pp. 124-139 (1981); Jackson, "The biochemistry of prothrombin activation" haemostasis and Thrombosis, 2nd Edition (Bloom et al., Eds.) Churchill Livingstone, Edinburgh, pp. 165-191 (1987) and Steinberg et al. "The activation of Factor X" Haemostasis and Thrombosis, First Edition (Colman et al., Eds.) Lippincott, Philadelphia, pp. 91-111 (1982).

Factor Xa plays a pivotal role in the blood coagulation cascade. Since it controls the coagulation cascade one step upstream from thrombin, a Factor Xa inhibitor may be a more efficient anticoagulant than those acting on thrombin. Heparin, which primarily blocks thrombin activity, is used most widely in conditions where thrombin production is responsible for the development of a thrombus. However, it is a heterogenous substance with many biological effects (Huisse et al.), thus necessitating the development of more specific and potent agents. The specificity and potency of antistasin have generated interest in its possible use as a more efficient clinical agent for the treatment of thrombotic diseases. Being a Factor Xa inhibitor, antistasin has an additional important biological function: as initially identified, it has strong anti-metastatic activity (Tuszinsky et al., J. Biol. Chem. 262 (1987) 9718-9723). There has been growing evidence that metastatic spread of tumor cells is mediated in part by abnormally stimulated blood coagulation (Gasic et al., Int. Rev. Exp. Path 29 (1986) 173-208), in which direct activation of Factor X by tumor cells has been suggested to be a major event (Falanga and Gordon, Biochemistry 24 (1985) 5558-5567). The pericellular deposition of fibrin is thought to be important in metastasis (Wood, J. Med. 5 (1974) 7-22) by providing tumor cells with a protective covering impermeable to the immune system (Gorelik et al., Int. J. Cancer 30 (1982) 107-112). Thus, the Factor Xa inhibitory activity of antistasin has been speculated to be the mechanism by which this protein reduces tumor spread.

Antistasin, a cysteine rich protein found in the salivary gland of the Mexican leech, Haementeria officinalis, has been shown to be a potent inhibitor of Factor Xa in the human blood coagulation cascade, Tuszynski et al.(1987); Nutt et al. J. Biol Chem 263: 10162-10167 (1988); Dunwiddie et al., J. Biol. Chem. 264: 16694-16699 (1989).

Therapeutic administration of large-size molecules such as antistasin is difficult. In general, bioabsorption of large molecules is less efficient than that for small molecules. Once present in the bloodstream, large molecules are susceptible to undesirable, inactivating chemical modification. They are therefore expected to have relatively short half-lives and short duration of activity. Therapeutic levels of large molecules in a patient's bloodstream are normally achieved by intravenous administration. For example, Harenberg et al., Seminars in Thrombosis and Hemostasis, vol. 15, no. 4 (1989) pp 414-423 compare bioavailability of normal heparin (approx. 12,000 molecular weight) with various lower molecular weight heparins (molecular weights ranging between 2,340 and 5,030). The smaller heparins are more effective following subcutaneous administration than normal heparin. Subcutaneous administration is not considered a suitable means for administering normal heparin. Applicants' subcutaneous means for administering the large molecule antistasin is shown to be a surprisingly effective method for inhibiting blood coagulation Factor Xa.

The present invention is a method for inhibiting blood coagulation Factor Xa which comprises administering to a patient an antistasin pharmaceutical composition according to a subcutaneous bolus injection regimen which achieves long-term therapeutically effective levels of antistasin in the blood stream. The composition and injection regimen enable a patient requiring Factor Xa blood coagulation inhibition to be effectively and conveniently treated.

## SUMMARY OF INVENTION

The invention is a method for inhibiting blood coagulation comprising administering to a patient by subcutaneous bolus injection once every 24-48 hours of a suitable amount of a pharmaceutical antistasin-containing composition sufficient to inhibit blood coagulation Factor Xa, wherein the composition is an antistasin/water solution, and wherein the amount of antistasin injected is about 0.05-5.0 milligram per kilogram patient body weight. The method achieves a steady-state plasma concentration of between about 10 nM and 100 nM over an extended period of time, e.g., up to 30 hours. By this method, blood clot formation time is increased. For example, blood clot formation time at 0.25 mg/kg is between 1.5 and 2-fold over antistasin-free blood clot formation time.

## DESCRIPTION OF THE FIGURES

Figure 1 - Ex vivo activated partial thromboplastin clotting time.
Figure 2 - Ex vivo prothrombin clotting time.
Figure 3 - In vitro activated partial thromboplastin clotting time with human, rhesus monkey, rabbit and dog plasma.
Figure 4 - Schematic representation of the expression plasmid for expressing antistasin cDNA in insect cells.

## DETAILED DESCRIPTION OF THE INVENTION

The amino acid sequence of antistasin administered in accordance with the method described below, is as follows: See ID Seq. No. 1

The invention comprises administering a protein, having the sequence shown above or having the sequence shown above with conservative amino acid substitutions, to a patient requiring inhibition of blood coagulation, by means of subcutaneous bolus injection once every 24-48 hours. The protein is administered in a suitable pharmaceutical composition such as a protein-water composition. The composition may have any suitable protein-water concentration, e.g., 1 mg/ml - 20 mg/ml, and pH, e.g., 7.4. Bolus injection of an amount of about 0.05-5 mg protein per kilogram patient body weight, preferably 1 mg/kg, achieves a pharmacologically effective plasma antistasin concentration over an extended period of time, e.g., up to 30 hours. The pharmacologically effective concentration ranges between about 5 nM and 100 nM, preferably between about 40 nM and 80 nM.

These proteins can, like many proteins, form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example acetic, glycolic, lactic, pyruvic, malonic, succinic, trifluroacetic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hyroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminium; and organic primary, secondary and tertiary amines, as for example, trialkylamines, including triethylamine, procaine, dibenzylamine, 1-ethenamine; N,N′-dibenzylethylenediamine, dihydroabietylamine, N-(lower)alkylpiperidine, and any other suitable amine.

Anticoagulant therapy is indicated for the treatment and prevention of a variety of thrombotic conditions, particularly coronary artery and cerebrovascular disease and thromboembolism resulting from disseminated intravascular coagulation. Those experienced in this field are readily aware of the circumstances requiring anticoagulant therapy. The term "patient" used herein is taken to mean mammals such as primates, including humans, sheep, horses, cattle, pigs, dogs, cats, rats, and mice.

The proteinaceous substance may be administered as injectable dosages of a solution or suspension of the substance in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water saline, aqueous dextrose and related sugar solutions, ethanol and glycols such as propylene glycol or polyethylene glycol are preferred liquid carriers, particularly for injectable solutions.

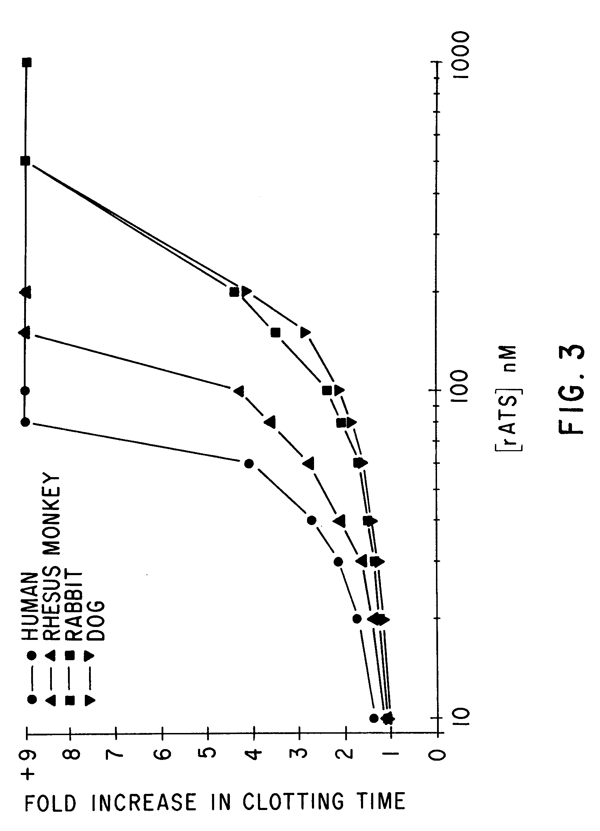

The data show that low dose subcutaneous administration of antistasin is a surprisingly effective means of inhibiting blood coagulation Factor Xa in primates. It is generally accepted that analysis of compounds in a primate model is strongly predictive of their effects in humans. Figure 3 shows the potency of antistasin in the APTT clotting assay with human, rhesus monkey, rabbit and dog plasmas. Potency is greatest with humans, and greater with rhesus monkeys than rabbits or dogs.

The protein may be used alone or in combination with other proteins. For example, the protein enhances the efficiency of tissue plasminogen activator-mediated thrombolytic reperfusion. The protein may be administered first following thrombus formation, and tissue plasminogen activator or other plasminogen activator is administered thereafter.

Anticoagulant proteins administered in accordance with the described procedure can be prepared by purification techniques described in Gasic et al., Cancer Res. 43 (1983) 1633-1636, and Gasic et al. Cancer Res. 44 (1984) 5670-5676, by cloning and expressing cDNA encoding antistasin as described in Han et al. Gene 75 (1989) 47-57, or by solid phase synthesis which is well known in the art, generally described in Merrifield, J. Am. Chem. Soc., 85, 2149 (1964), Houghten, Proc. Natl. Acad. Soc., 82, 5132 (1985) and Rivier et al., U.S. Patent No. 4,244,946. These proteins and proteins having conservative amino acid substitutions are suitable for administration in accordance with the procedure of the invention.

Antistasin may be prepared from two cDNA clones, (lambda 0-13 and lambda 5C-4)(See Han et al., Gene vol. 75 (1989) 47-57). The deduced sequences are shown in Sequence ID Nos. 1 and 6. For preparation of antistasin via cloning and expression of cDNA, all restriction endonucleases, DNA modifying enzymes, reverse transcriptase and terminal deoxynucleotidyl transferase were obtained from Boehringer Mannheim, New England Biolabs, Bethesda Research Laboratories or PL-Pharmacia, phage lambda packaging extract and rabbit reticulocyte lysate from Promega Biotec, radio-isotopes from Amersham, bovine factors Xa and V from Enzyme Research Labs, H-D-henylalanyl-L-pipecolyl-L-arginine-p-nitroanilide dihydrochloride (S2238) from Helena Labs, and nylon membranes (GeneScreen Plus*) from New England Nuclear.

Salivary glands were dissected from H. officinalis, dropped into liquid nitrogen, and stored at -70°C until use; poly(A)+RNA was extracted from the gland (Han et al. Biochemistry 26 (1987) 1617-1625). DNA was obtained from adult gonads or whole young animals as described (Maniatis et al. Molecular Cloning. A Laboratory Manual Cold Spring Harbor Laboratory, Cold Spring Harbor, NY (1982)). Northern and Southern blot analyses of RNA and DNA were preformed according to standard procedures (Maniatis et al., 1982).

A lambda gt22cDNA expression library was constructed from leech salivary gland poly(A)+RNA according to Han and Rutter (Genetic Engineering vol. 10, Plenum Press, NY (1988)). Briefly, the poly(A)+RNA was converted into first-strand cDNA using a primer-adapter that contained a NotI site, SP6 promoter and a stretch of dT's: See Seq. ID No. 2 The resultant first strand cDNA was tailed with dG [about 15 nt] and converted into ds cDNA using a second-strand primer-adapter that contained a Sa1I site, T7 promoter and stretch of dC's: See Seq. ID No. 3. The ds cDNA was cleaved with NotI and Sa1I and cloned into the corresponding sites of lambda gt22. To screen the library, two oligos were synthesized on the basis of a preliminary amino acid sequence of antistasin, now known to reside between aa residues 70 and 107 of the mature protein (Nutt et al. J. Biol. Chem. 263 (1988) 10162-10167). They were: See Seq. ID Nos. 4 and 5. These two oligos have 18 bp of complementary overlap at each 3'-end by which they were annealed and repaired with Po1Ik in the presence of the four [$\alpha$-$^{32}$P]dNTPs. This generated a ds probe that was 114 bp long and 256-fold degenerate with a specific activity of 5 x 10$^9$ cpm/$\mu$g. DNA filters were hybridized at 42°C in 20% formamide, 6 x SSC (1 x = 150 mM NaCl, 15 mM Na$_3$·citrate, pH 7.0) and washed in 42°C in 0.2 x SSC/0.1% SDS for 30 minutes. The library also was screened (Young and Davis, Science 222 (1983) 778-782) with rabbit antibodies to antistasin (Tuszinsky et al. J. Biol. Chem. 262 (1987) 9718-9723).

Both sense and antisense mRNA were synthesized directly from the isolated phage DNAs which were positive using either oligo or antibody probes. In vitro transcription was performed (Melton et al. Nucleic Acids Res. (1984) 7035-7057), and the capped transcripts were translated in vitro in the presence of [$^{35}$S]Met using a rabbit reticulocyte lysate (Pelham and Jackson Eur. J. Biochem. 67 (1976) 247-266). The labelled protein was immunoprecipitated and resolved by 0.1% SDS-15% PAGE.

The nucleotide sequence of cDNA inserts was determined (Chen and Seeburg, DNA 4 (1985) 165-176) either directly on lambda DNA or after subcloning into pUC18.

An antistasin cDNA was reconstructed into an expression plasmid using two sets of synthetic oligos. Briefly, the cDNA insert from lambda 0-13 was cut with AvaII + HindIII, then ligated to oligos that generated Bg1II termini. This rebuilt cDNA then was inserted into the BamHI site of baculovirus expression vector pAc373 (Smith et al, Mol. Cell. Biol. 3 (1983) 2156-2165), in which expression is driven by the viral polyhedrin promoter. A recombinant baculovirus was isolated after cotransfection of the resultant plasmid (pBD88-004-05) with AcNPV DNA into Sf9 cells by the calcium phosphate method (Summers and Smith, A Manual of Methods for Baculovirus Vectors and Insect Cell Culture Procedures. Texas Agricultural Experiment Section Bulletin, No. 1555 (1987)).

Antistasin in cell-free medium was adsorbed onto heparin-sepharose and eluted with 0.55 M NaCl (Tuszinsky et al. 1987). Proteins in the eluate were concentrated using a Centricon-10® membrane, resolved by 0.1% SDS-11.5% PAGE, electroblotted onto nylon membranes, and incubated with a 1:250 dilution of guinea pig anti-antistasin, as described (Towbin et al. Proc. Natl. Acad. Sci. USA 76 (1979) 4350-4354).

In the lambda gt22cDNA expression library construction, each cDNA was flanked by SP6 and T7 polymerase piomoters and then inserted into the 3' end of the lacZ gene in one orientation. This library was screened using several degenerate oligo probes that were designed on the basis of a selected amino acid sequence from an antistasin peptide generated by V8 protease cleavage (Nutt et al. 1988). Sequence degeneracy in each probe was reduced by allowing G/T mismatches and employing codon usage published for Drosophila (Maruyama et al. Nucleic Acids Res. 14 (1986) 151-197). Northern blot analysis was performed to test the hybridization specificities of these probes and to tentatively determine the size of antistasin mRNA. Upon hybridization to salivary gland RNA, one probe that was 114 bp long and 256-fold degenerate detected a single RNA species of ca. 750 nt. An RNA band of the same size was detected using a sequenced cDNA probe in later experiments.

Forty positive clones out of approx. $2 \times 10^6$ plaques from the lambda gt22 library were identified using the 114-bp oligo probe. By screening the same number of recombinant plaques with rabbit antibodies to antistasin, an additional ten positive clones were identified, three of which hybridized with the oligo probe. Some of the candidate antistasin clones were tested in vitro for their ability to encode immunologically reactive proteins. Transcription of two purified recombinant phage DNAs (lambda 0-13 and lambda 5C-4) by T7 RNA polymerase and subsequent translation in a rabbit reticulocyte lysate produced a 17-kDa protein that was precipitated specifically by these antibodies. The addition of synthetic antisense RNA (produced by transcription of the same phage DNA with Sp7 polymerase) to the in vitro translation reaction using poly(A)+RNA elimiated detectable antistasin.

Nucleotide sequences of two cDNA inserts (lambda 0-13 and lambda 5C-4) that produced immunoreactive proteins in vitro are presented in Han et al., p. 52. Antistasin mRNA, as deduced from cDNA clone lambda 5C-4, contains an uninterrupted ORF of 411 nt. This ORF is preceded by a 59-nt AT-rich (95%) 5'-untranslated sequence and followed by a 195-nt 3'-untranslated region which consists of TA repeats occasionally interrupted by G or C residues. A hexanucleotide AATAAA consensus signal for polyadenylation is found 14 nt before the poly(A)+tract. Thus, this cDNA encodes an mRNA sequence whose size is consistent with the 750-nt size in Northern blot analysis, assuming the length of the poly(A)+tract as ca. 100 nt.

Translation of the ORF generated a 137-aa sequence that matches very closely with the recently resolved sequence of the mature protein (Nutt et al. 1988). The N-terminal Gln residue of mature antistasin is preceded by an 18-aa pre-peptide beginning with two consecutive Met residues. These 18-aa residues contain a hydrophobic core of 13 aa flanked by two charged residues typical of a signal peptide (Han et al., p. 52).

The amino acid sequence determination of antistasin indicated the existence of variants at two residues: aa 5 (Arg/Gly) and aa 35 (Met/Val) (Nutt et al. 1988). Direct nucleotide sequence analysis of independent cDNA clones confirmed the variation at aa 35 and revealed additional variations at residues 30 (Gly/Glu) and 54 (Arg/Ile).

The complexity of the antistasin locus in the leech genome was studied by Southern blot analysis. Leech genomic DNA was digested with restriction endonucleases and analyzed by hybridization with the cDNA insert from lambda 0-13. Five restriction endonucleases, individually or in combination with a four-base cutter, generated a single-band pattern in most cases. This result suggests that the leech genome contains a single copy of the antistasin gene.

The complete antistasin cDNA sequence was modified at the 5'- and 3'- ends, and the gene cassette was inserted into the baculovirus expression vector pAc373 to yield pBD88-004-5. This vector contains a polyhedrin promoter, polyadenylation signal and sufficient flanking viral sequences for homologous recombination with AcNPV. The plasmid and viral DNAs were cotransfected into the insect cell line Sf9 for production and purification of a recombinant virus. Antistasin activity was measured by determining the percent inhibition of the conversion of prothrombin to thrombin by factor Xa. Antifactor Xa activity was detected in culture supernatants at 5 days post-transfection. A 1:4 dilution of growth medium exhibited inhibition of factor Xa activity as compared to the medium taken from mock-transfected cultures. This inhibition was abrogated by preincubation of the sample with a 1:50 dilution of guinea pig anti-antistasin serum. Based on activity, the level of transiently expressed antistasin in the culture medium was 40 ng/ml. Immunoblot analysis was performed on the antistasin produced from insect cells transfected with three distinct recombinant baculoviruses. A 15-kDa protein was detected by antistasin-specific antibodies. This protein was indistinguishable in electrophoretic mobility from the native protein but migrated slightly more rapidly than the protein derived from in vitro translation of RNA, consistent with the processing of the 17-aa prepeptide.

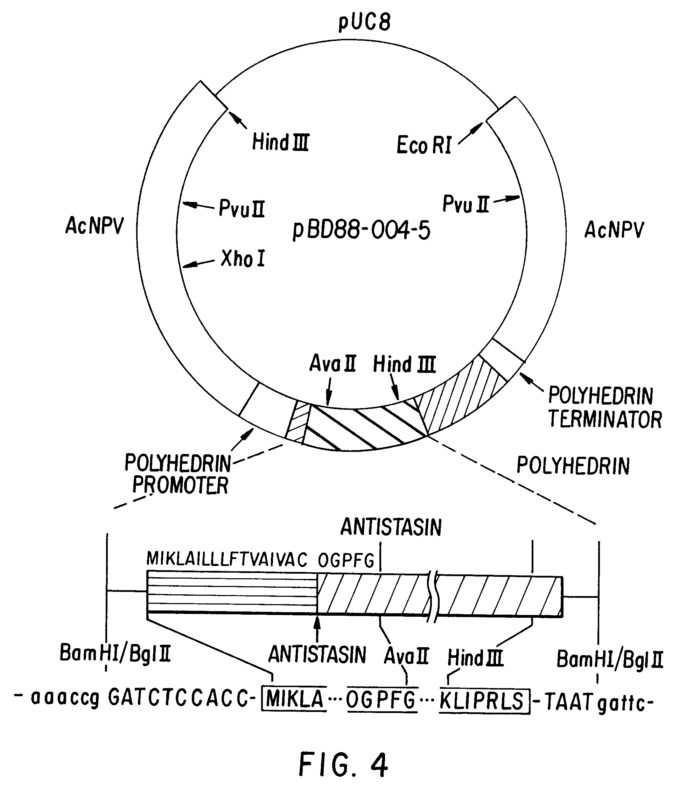

Figure 4 is a schematic representation of the expression plasmid for expressing antistasin cDNA in insect cells. The upward arrow indicates the presumed cleavage site recognized by the signal peptidase. Two restriction sites (AvaII and HindIII) that were used to reconstruct the 5'- and 3'- ends of the complete antistasin ORF are indicated. Parts of the nucleotide sequences in the 5'- and 3'- oligos are shown. The nucleotide sequence in the lower case represents vector sequences at the BamHI cloning site.

◩    polyhedrin transcriptional promoter and terminator

▤    antistasin pre-peptide

■    antistasin mature protein

▨    polyhedrin flanking sequences

☐    AcNPV flanking sequences

———    pUC8

Example 1

A 14.5 mg/ml antistasin stock solution in water quantitated in duplicate by direct amino acid composition analysis was the source of material used in this study. Antistasin was administered subcutaneously to two conscious Rhesus monkeys at a position roughly midway between the shoulder blades on the upper back. A dose of 1 mg/kg was achieved by injection of 0.175 mls (2.54 mg) of the stock solution to monkey #89-041 and 0.195 mls (2.83 mg) to monkey #89-043. Blood samples were collected in 3.8% sodium citrate (final concentration of 0.38% sodium citrate) before administration of antistasin and at 0.5, 1, 2, 4, 6, 8, 12, 24 and 30 hours following antistasin administration. Plasma was prepared by centrifugation and stored at -70°C. prior to analysis. Ex vivo clotting times were determined using both the activated partial thromboplastin time and prothrombin time clotting assays.

Activated Partial Thromboplastin Time (APTT)

Blood samples were collected in sodium citrate (final concentration of 0.38%), and plasma was prepared by centrifugation for 10 to 15 minutes. Clotting times were determined in duplicate for each sample on a MLA Electra 700 instrument. Briefly, 100 ul of plasma was added directly to a cuvette placed in the instrument and incubated for 5 minutes at 37°C. Following the incubation, 100 ul of the Actin-Activated Cephaloplastin Reagent were added followed by 100 ul of a-0.02 M $CaCl_2$ solution and the clotting time was measured in seconds.

Prothrombin Time (PT)

Plasma samples were prepared as described above. 100 ul of plasma was added directly to a cuvette placed in the MLA 700 and incubated for 3 minutes at 37°C. Following incubation, 200 ul of the Thromboplastin C Reagent (rabbit brain thromboplastin with calcium) was added and the clotting time was measured in seconds.

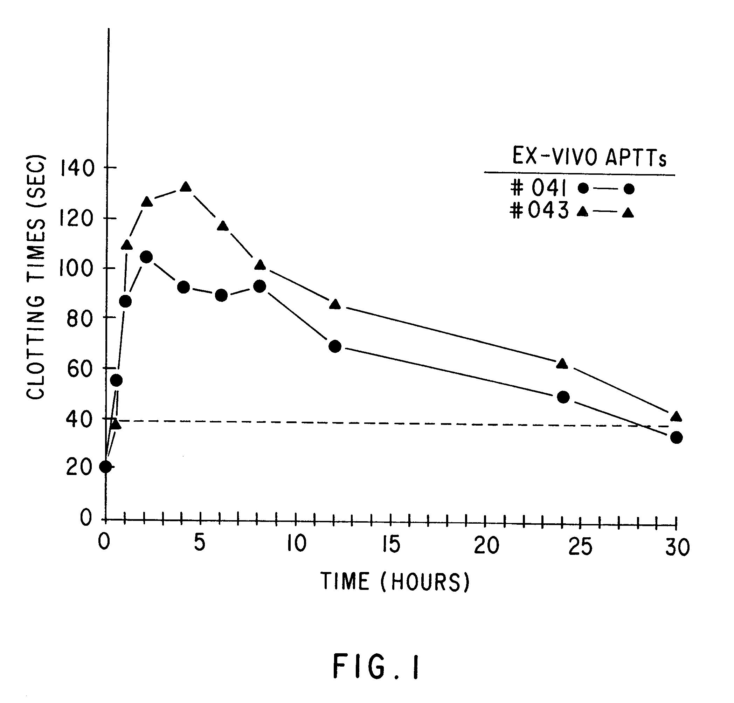

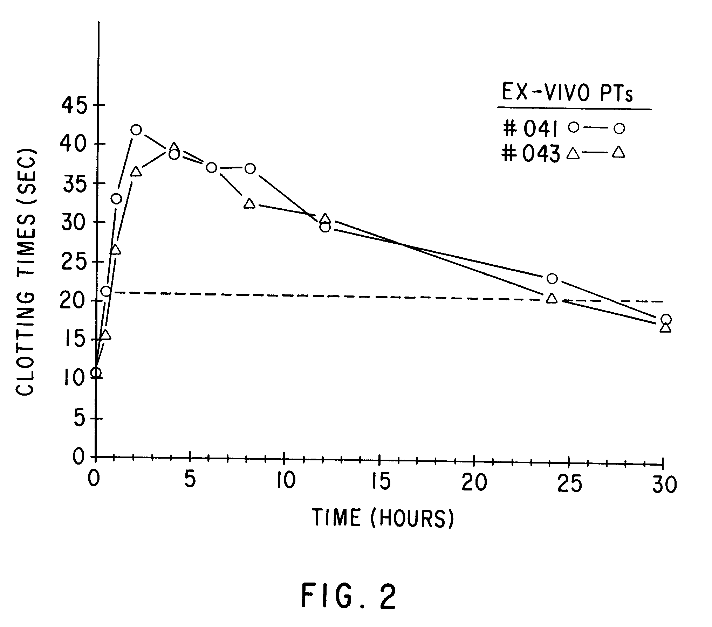

Ex vivo clotting time data illustrated in Figure 1 and Figure 2 shows that elevated antistasin levels are achieved for a long period of time. Figures 1 and 2 show that the peak prolongation of APTT and PT clotting time occurs about 2-4 hours post-bolus time. The increase in APTT clotting time at 30 hours post-bolus time is 1.86-fold. The increase in PT clotting time, at 30 hours post-bolus time is 1.69-fold.

Elevated antistasin levels are quite apparent at 30 minutes post-bolus time as indicated by a 2.23-fold and 1.81-fold prolongation of the APTTs for monkeys #89-041 and #89-043, respectively. The highest antistasin levels are reached between 2 and 4 hours post-bolus time and are associated with a peak prolongation of the APTT and PT clotting times by 5.7-fold and 3.8-fold, respectively. These clotting time increases correspond to peak plasma antistasin concentrations of 100-120 nM. After 30 hours the plasma antistasin concentrations are 25-30 nM. Ex vivo recombinant antistasin concentrations were determined from a standard curve of APTT time versus recombinant antistasin concentration determined in control rhesus plasma.

These data indicate that antistasin at a relatively modest dose of 1 mg/kg is sufficiently absorbed from a subcutaneous bolus injection administration to obtain clotting time increases well above those deemed necessary for efficacy with standard heparin in a clinical setting (1.5 to 2-fold elevated above control). Furthermore, the levels of antistasin obtained in plasma are well maintained over the full time course of thirty hours.

SEQUENCE DESCRIPTION: SEQ ID NO: __1__

SEQ ID NO: __1__

```
         1            5                    10                    15
       Glu Gly Pro Phe Gly Pro Gly Cys Glu Glu Ala Gly Cys Pro Glu

                       20                    25                    30
       Gly Ser Ala Cys Asn Ile Ile Thr Asp Arg Cys Thr Cys Ser Glu

                       35                    40                    45
       Val Arg Cys Arg Val His Cys Pro His Gly Phe Gln Arg Ser Arg

                       50                    55                    60
       Tyr Gly Cys Glu Phe Cys Lys Cys Arg Leu Glu Pro Met Lys Ala

                       65                    70                    75
   Thr Cys Asp Ile Ser Glu Cys Pro Glu Gly Met Met Cys Ser Arg

                       80                    85                    90
   Leu Thr Asn Lys Cys Asp Cys Lys Ile Asp Ile Asn Cys Arg Lys

                       95                   100                   105
   Thr Cys Pro Asn Gly Leu Lys Arg Asp Lys Leu Gly Cys Glu Tyr

                      110                   115                   119
   Cys Glu Cys Arg Pro Lys Arg Lys Leu Ile Pro Arg Leu Ser OH
```

SEQUENCE DESCRIPTION: SEQ ID NO: __2__

SEQ ID NO: __2__

5' AATTCGCGGC CGCCATACGA TTTAGGTGACA CTATAGAATT TTTTTTTTTT TTT 3'   53

SEQUENCE DESCRIPTION: SEQ ID NO: __3__

SEQ ID NO: __3__

5' AATTCGTCGA CAATACGACT CACTATAGGG AGACCCCCCC CCCCCCCC 3'   48

SEQUENCE DESCRIPTION: SEQ ID NO: __4__

SEQ ID NO: __4__

5' GGCATGATGT GCAGCCGGGT GACTAATAAG TGCGATTGCA AGATTGATAT TAATGCCGCA   60
              T         T                  T        T

  AG 3'   62

SEQUENCE DESCRIPTION: SEQ ID NO: _5_

SEQ ID NO: _5_

```
5' TTCGCAGTAT TCGCAGCCCA GCTTATCGCG CTTCAGGCCA TTCGGGCAAG TCTTGCGGCA    60
        A         A          A          A          A

    ATTAATATC 3'                                                        69
```

SEQUENCE DESCRIPTION: SEQ ID NO: _6_

SEQ ID NO: _6_

```
  1              5                   10                  15
Glu Gly Pro Phe Gly Pro Gly Cys Glu Glu Ala Gly Cys Pro Glu

                 20                  25                  30
Gly Ser Ala Cys Asn Ile Ile Thr Asp Arg Cys Thr Cys Ser Gly

                 35                  40                  45
Val Arg Cys Arg Met His Cys Pro His Gly Phe Gln Arg Ser Arg

                 50                  55                  60
Tyr Gly Cys Glu Phe Cys Lys Cys Ile Leu Glu Pro Met Lys Ala

                 65                  70                  75
Thr Cys Asp Ile Ser Glu Cys Pro Glu Gly Met Met Cys Ser Arg

                 80                  85                  90
Leu Thr Asn Lys Cys Asp Cys Lys Ile Asp Ile Asn Cys Arg Lys

                 95                  100                 105
Thr Cys Pro Asn Gly Leu Lys Arg Asp Lys Leu Gly Cys Glu Tyr

                 110                 115            119
Cys Glu Cys Arg Pro Lys Arg Lys Leu Ile Pro Arg Leu Ser OH
```

EP 0 468 764 A1

**Claims**

1. A pharmaceutical composition adapted for subcutaneous administration by bolus injection, comprising a pharmacologically acceptable concentration of antistasin in water.

2. A composition as claimed in Claim 1 wherein the concentration of antistasin in water is in the range of from 1mg/ml to 20mg/ml.

3. A composition as claimed in Claim 1 or Claim 2 in unit dosage from wherein the dose is between about 0.05mg and about 5mg of antistasin per kilogram of patient body weight.

4. A composition as claimed in any of claims 1 to 3 adapted to maintain a steady-state plasma antistasin concentration of between about 1nM and about 100nM over a 30 hour period following administration.

5. A composition as claimed in Claim 4 adapted to maintain a steady-state plasma antistasin concentration of between about 40nM and about 80nM over a 30 hour period following administration.

FIG. 1

FIG. 2

FIG. 3

EP 0 468 764 A1

FIG. 4

European Patent
Office

**EUROPEAN SEARCH REPORT**

Application Number

EP    91 30 6739

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-263608 (THE CONTRIBUTORS TO THE PENNSYLVANIA HOSPITAL) <br> * page 3, lines 3 - 5 * <br> * page 4, lines 20 - 24 * <br> --- | 1-5 | A61K37/64 <br> C07K15/00 <br> A61K35/62 |
| Y | EP-A-352903 (YISSIUM RESEARCH DEVELOPMENT COMPANY OF THE HEBREW UNIVERSITY) <br> * page 4, line 4 * <br> --- | 1-5 | |
| Y | EP-A-346894 (MERRELL DOW PHARMACEUTICALS) <br> * page 4, lines 2 - 5 * <br> * page 4, line 12 * <br> --- | 1-5 | |
| Y | EP-A-209061 (HOECHST) <br> * page 13, line 28 * <br> * page 13, line 36 * <br> --- | 1-5 | |
| A | US-A-4588587 (GASIC G.J.) <br> * the whole document * <br> --- | 1-5 | |
| A,P | EP-A-404055 (MERRELL DOW PHARMACEUTICALS) <br> * the whole document * <br> ----- | 1-5 | **TECHNICAL FIELDS SEARCHED (Int. Cl.5 )** <br><br> A61K <br> C07K |

The present search report has been drawn up for all claims

| Place of search | Date of completion of the search | Examiner |
|---|---|---|
| BERLIN | 06 NOVEMBER 1991 | AVEDIKIAN P.F. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)